# EUROPEAN PATENT APPLICATION

(11) **EP 3 085 317 A1**
(43) Date of publication of application: **26.10.2016**
(21) Application number: 15164776.5
(22) Date of filing: 23.04.2015
(51) Int. Cl.: A61B 17/435, A61M 1/00

(54) **OOCYTE RECOVERY APPARATUS AND METHOD OF ITS USE**

(71) Applicant: Shivani Scientific Industries Private Limited, 401104 Mumbai (IN)
(72) Inventor: Kale, Ravikant, 401104 Mumbai (IN); Modi, Ashish, 401104 Mumbai (IN)
(74) Representative: Nordmeyer, Philipp Werner

(57) **Abstract**

The present disclosure discloses an apparatus and method of using such apparatus for recovering oocyte from patient's uterus. The present disclosure provides hassle-free and user-interactive experience to the user using the apparatus during the oocyte recovery process. The apparatus comprises a profile control module, a user interface module, and proportional control module. The profile control module defines a plurality of profiles. Further, each of the plurality of profiles comprises a plurality of attributes having a plurality of values. The user interface module enables a user to select a profile from the plurality of profiles and a value corresponding to each attribute of the profile selected. Further, proportional control module generates a vacuum level, via a vacuum generating unit, based upon the profile and the value select by the user. Further, the vacuum level generated may be proportionally controlled based on pressure applied on foot pedal.

## Description

### TECHNICAL FIELD

The present disclosure described herein, in general, relates to an oocyte recovery apparatus used in In-vitro fertilization (IVF) type treatment.

### BACKGROUND

In-vitro fertilization (IVF) also known as an assisted reproductive technique is considered as a major treatment process for infertility. The treatment process involves collecting follicle fluid from a patient's ovaries into a collecting unit such as a glass container, beaker, and test-tube etc. The follicle fluid collected in the collecting unit may contain an oocyte (i.e., an egg). Once the follicle fluid is collected with the oocyte, the oocyte is fertilized outside the patient's body to form a fertilized egg (zygote). Further, the fertilized egg (zygote) is transferred into the patient's uterus for establishing a successful pregnancy. The collection of oocyte, however, requires greater skill and expertise so that no damage is being done to the oocyte (egg).

In general, for the collection of the oocyte, an oocyte aspirator may be used by a physician or an assistant assisting the physician. The oocyte aspirator may comprise a needle, a vacuum generating unit, a collecting unit, and an auxiliary tube. The needle, from one end, is connected with the vacuum generating unit through the auxiliary tube. When the needle is inserted into the patient's ovaries for collecting the follicle fluid containing the oocyte, an appropriate amount of vacuum needs to be generated by the vacuum generating unit for safely collecting the follicle fluid into the needle and further collecting the follicle fluid into the collecting unit through an auxiliary tube. Specifically, during extraction of the follicle fluid, an appropriate amount of vacuum level may required to be generated in a manner such that it does not harm/damage the oocyte. Thus, there may be a need to have a control over vacuum level settings inside the needle which regulates the generation of the appropriate amount of vacuum as required for the extraction of the oocyte.

The control over the vacuum level settings may vary according to variable conditions in the patient's body. Any over-pressure or under-pressure situations generated due to the vacuum level settings may cause damages to the oocyte. Moreover, the oocyte aspirators available today come with standard vacuum level settings which may be predefined by manufacturers producing the oocyte aspirators. This gives a limited scope to the physician in regulating and controlling of the vacuum level settings in order to generate desired level of the vacuum for the extraction of the oocyte.

Conventionally, the collection of the oocyte and the controlling of the vacuum level settings are being performed manually by the physician. The physician performing such operations needs to be trained in order to handle and operate the oocyte aspirator. The physician may further have to perform the tasks with proper co-ordination in order to collect the oocyte from the patient's ovaries, successfully. Any mismatch in the co-ordination may result not only in an unsuccessful collection, but may also cause serious injuries to the patient.

### SUMMARY

This summary is provided to introduce aspects related to an oocyte recovery apparatus for providing user-friendly and user-interactive experience during extraction and handling an oocyte from patient's ovaries and the concepts are further described below in the detailed description. This summary is not intended to identify essential features of disclosure nor is it intended for use in determining or limiting the scope of the disclosure.

In one implementation, an oocyte recovery apparatus (hereinafter referred as "apparatus") and method of using such apparatus is disclosed. The apparatus provides a proper control over a vacuum level required for the oocyte recovery from the patient's ovaries. The apparatus comprises a needle, a collecting unit, a vacuum generating unit, an auxiliary tube, and a foot pedal. The apparatus further comprises a memory coupled with a processor, wherein the processor executes a plurality of modules stored in the memory. The plurality of modules may comprise a profile control module, a user interface module, and a proportional control module. The profile control module may define a plurality of profiles. Further, each of the plurality of profiles may comprise a plurality of attributes having a plurality of values. Each profile is indicative of appropriate vacuum level settings required for generating a desired level of vacuum through the vacuum generating unit. The profiles may be configured based on past experience and performance of vacuum level settings that would facilitate the generation of the appropriate level of the vacuum as desired for the retrieval and thereby recovery of follicle fluid containing the oocyte. Further, the user interface module may enable a user to select a profile from the plurality of profiles and a value corresponding to each attribute of the profile selected. Further, the proportional control module may generate the vacuum level, via the vacuum generating unit, based upon the profile and the value selected by the user. Further, the vacuum level generated may be proportionally controlled based on a pressure applied on the foot pedal. The operation performed on the foot pedal may be directly sensed by the proportional control module. Also, the operation performed on the foot pedal may activate or de-active the functioning of the vacuum generating unit. Based on the pressure applied or released on the foot pedal, amount of vacuum level may be increased or decreased. Thus, the vacuum level may be gradually increased or decreased in the needle with the help of the foot pedal. The gradual increase or decrease in the vacuum level may enable the user to safely extract the follicle fluid into the needle and then transfer it into the collecting unit. Hence, the above discussed methodologies may give the user a hassle-free experience during the recovery of the oocyte in the safer manner.

### BRIEF DESCRIPTION OF THE DRAWINGS

The detailed description is described with reference to the accompanying figures. In the figures, the left-most digit(s) of a reference number identifies the figure in which the reference number first appears. The same numbers are used throughout the drawings to refer like features and components.
Figure 1 illustrates a functional block diagram 100 of the apparatus, in accordance with an embodiment of the present disclosure.
Figure 2 is an example illustrating a profile along with its plurality of attributes and values of the attributes displayed on the user interface module, in accordance with an embodiment of the present disclosure.
Figure 3 illustrates a method for controlling vacuum level required for oocyte recovery, in accordance with an embodiment of the present disclosure

### DETAILED DESCRIPTION

According to various aspects of present disclosure, an apparatus and method of using such apparatus is disclosed. The purpose of the apparatus is to provide a hassle-free and user-interactive method for technicians or operators using the apparatus. Hereinafter, the term the "technicians" or the "operators" may be collectively referred as "user". In-vitro fertilization (IVF) is a known treatment for infertility. In the treatment process, the follicle fluid is extracted or recovered from the patient's ovaries and collected in a collecting unit such as test-tube or other type of receptacle. The follicle fluid collected in the collecting unit contains an oocyte i.e., an egg which is used for fertilization, wherein the fertilization may take place in the collecting unit. Thus, after the fertilization, a fertilized egg (zygote) i.e., a diploid cell is produced, wherein the zygote is transferred back into patient's uterus with an intention to establish a successful pregnancy.

During the extraction of the follicle fluid, the apparatus plays an important role. The apparatus may comprise a needle, a collecting unit, a vacuum generating unit, and an auxiliary tube. While conducting the IVF treatment, one of a major treatment process involved is aspiration i.e., extraction of the follicle fluid into the collecting unit. The aspiration process may be initiated by inserting the needle (from its one end) through patient's vaginal wall into the patient's ovaries for extracting the follicle fluid and transferring said follicle fluid into the collecting unit of the apparatus. For extracting the follicle fluid into the needle, an appropriate amount of vacuum may be required to be generated. The vacuum may be generated by the vacuum generating unit using a foot pedal coupled of the apparatus.

During the treatment, the user may operate the needle's movement under ultrasound guidance after the needle is inserted into the patient's body. While operating the needle, the user may have to ensure about safer recovery of the follicle fluid without causing any harm to the internal organs of the patient's body. Apart from operating the needle, the user may also have to monitor and control the vacuum generation in order to generate a desired level of vacuum inside the needle for extracting the oocyte safely. In performing these functions at the same time, the user may lose his/her concentration which may further result in unsuccessful extraction of the follicle fluid which further may cause serious injuries in the patient's internal organs. The present disclosure enables to monitor and control the generation of the appropriate vacuum via the foot operated pedal, as explained in detail as below:

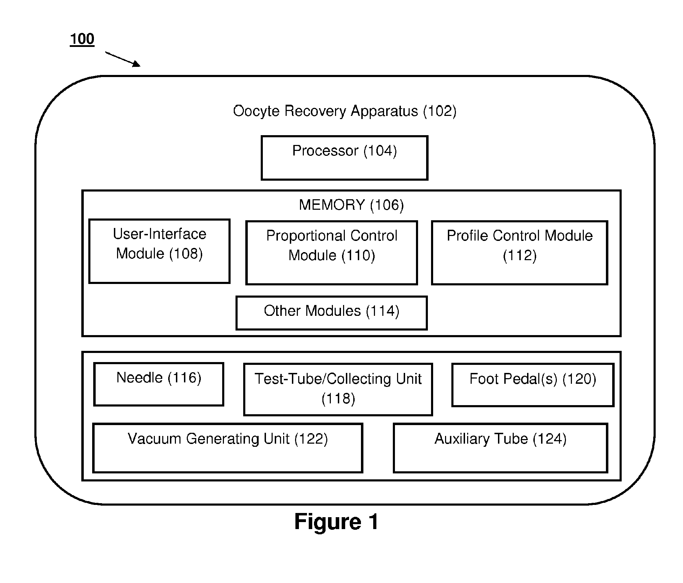

Referring to Figure 1 a functional block diagram 100 of the apparatus 102 comprising various modules are shown, in accordance with an embodiment of the present disclosure. In one embodiment, the apparatus 102 comprises a processor 104, and a memory 106. The memory 106 comprises various modules including a user-interface module 108, a proportional control module 110, a profile control module 112, and other modules 114. The apparatus 102 further comprises other components like needle 116, collecting unit 118, a foot pedal 120, a vacuum generating unit 122, and an auxiliary tube 124 for performing oocyte recovery from patient's uterus.

According to some embodiments, the processor 104 may be implemented as one or more microprocessors, microcomputers, microcontrollers, digital signal processors, central processing units, state machines, logic circuitries, and/or any devices that manipulate signals based on operational instructions. Among other capabilities, the at least one processor 104 is configured to fetch and execute computer-readable instructions or modules stored in the memory 106. According to embodiments of present subject matter, a processor 104 may execute the various modules (the user-interface module 108, the proportional control module 110, the profile control module 112, and the other modules 114) stored in the memory 106 of the apparatus 102 to enable the apparatus 102 to perform follicle fluid recovery according to various embodiments of the present subject matter.

Further, the memory 106 may include any computer-readable medium or computer program product known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, a compact disks (CDs), digital versatile disc or digital video disc (DVDs) and magnetic tapes. The memory 106 may include various modules i.e., the user-interface module 108, the proportional control module 110, the profile control module 112, and the other modules 114.

For recovering/extracting the follicle fluid, the user may insert the needle 116 from one end through a vaginal wall of the patient under ultrasound guidance. The needle 116 is inserted into an ovarian follicle to recover one or more oocytes hereinafter referred to as "oocyte", in a manner such that it does not injure internal organs located between the vaginal wall and ovary of the patient's body. To safely recover the oocyte, the user may operate the foot pedal 120. According to embodiments of present disclosure, the profile control module 112 may define a plurality of profiles. Further, each of the plurality of profiles may comprise a plurality of attributes having a plurality of values. Thus, there may be 'n' number of attributes with 'n' number of values corresponding to each attribute. Further, the profile control module 112 may be configured to operate the vacuum generating unit 122 in such a manner that the vacuum generating unit 122 may generate different levels of vacuum required by the user at a certain time instance. The different levels of the vacuum may be generated via the profile control module 112 by controlling valves (solenoid valves) of the vacuum generating unit 122.

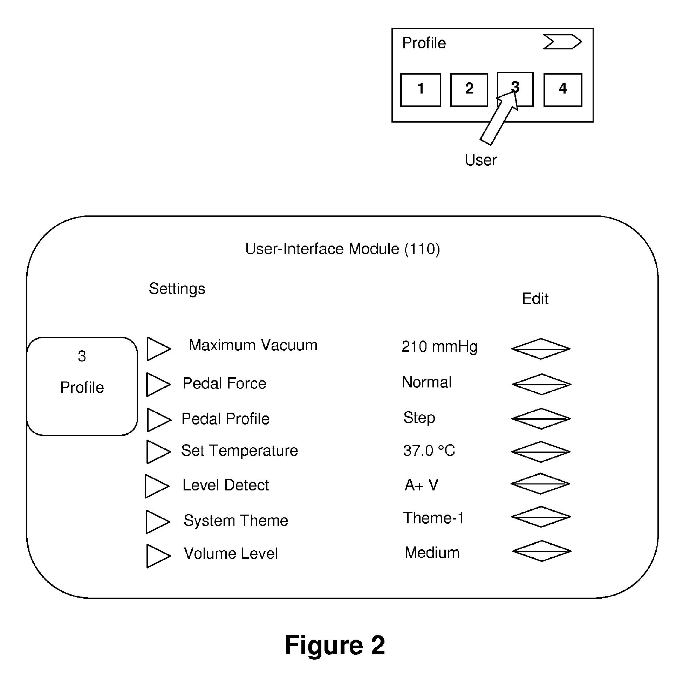

Further, the user interface module 108 of the apparatus may give access or may enable the user to a select profile from the plurality of profiles and a value corresponding to each attribute of the profile selected. Each profile may comprise a predefined vacuum level which may be gradually achieved when the user applies the pressure on the foot pedal 120. The various profiles can be seen from the figure 2.

Referring to figure 2 is example illustrating a profile along with its plurality of attributes and values of the attributes displayed on the user interface module 108, in accordance with an embodiment of present disclosure. From the figure 2, it can be seen that the user may select one of a profile (Profile 3) from plurality of profiles (Profile 1, Profile 2, Profile 3, Profile 4) displayed through the user-interface module 108. In the present example, the user has selected "Profile 3". It may be noted that, according to embodiments of the present disclosure, there may be 'n' number of profiles provided by the apparatus 102 which may be selected by the user. Upon selecting the profile, the profile control module 112 may be further configured for listing plurality of attributes and their values of the profile selected by the user. Further, the plurality of attributes may be displayed under "Settings" on the user-interface. The user may further interact through the user-interface module 108 and choose/select/set different values of the plurality of attributes displayed corresponding to the profile selected. The selected values of the attributes may be changed/altered/modified at any instance of time during the recovery process of the oocyte. Further, the user-interface module 108 may include a variety of software and hardware interfaces, for example, a web interface, a graphical user interface, and the like.

For example, for one of the attribute i.e., "Maximum Vacuum", the user may select its value as "210 mmHg" as can be seen in the figure 2. Similarly, the user may also select the value as "Normal" for another attribute i.e., "Pedal Force". According to embodiments of the present disclosure, there may be different types of values for the attribute "Pedal Force" which may be provided for being selected by the user, wherein the different types of values may be Normal, Light, Standard, and Heavy. Yet another value which may be selected by the user is "Step" corresponding to the attribute "Pedal Profile". According to embodiments of the present disclosure, there may be different types of values for the attribute "Pedal Profile" which may be also provided for selection by the user. The different values for the attribute "Pedal Profile" may be Rapid, Step, Cruise control, and Proportional.

Further, the proportional control module 110 may generate the vacuum level, via the vacuum generating unit 122, based upon the profile and the value of the attributes selected by the user. Further, the vacuum level generated may be proportionally controlled based on a pressure applied on the foot pedal 120. The user may operate the foot pedal by applying and releasing pressure upon the foot pedal 120. Based on the pressure applied and released on the foot pedal 120, the vacuum level may be gradually increased and decreased. Thus, the proportional control module 110 may control the vacuum level generated from the vacuum generating unit by sensing the action performed on the foot pedal 120.

Further, the proportional control module 110 may be also configured to generate the vacuum level based on weight and height of the user. The user may be grouped in different categories based on combination of their weight and height. According to embodiments of present subject matter, the grouping may be done based on other features of the user including the weight and the height. According to one embodiment, the different groups may be G1, G2, and G3. The group G1 refers to the users falling under the weight range between 20kg-40kg, the group G2 refers to the users falling under the weight range between 41kg-60kg, and the group G3 refers to the users having weight more than 60kg. Depending on the weight of the user, the pressure applied on the foot pedal 120 may vary, and therefore may result in different levels of vacuum generation. For example, the pressure applied on the foot pedal 120 by a user of the group G1 (having weight 22 kg) may be pretty less than the pressure applied by another user of the group G3 (having weight 63 kg). Therefore to overcome such situations, the proportional control module 110 is configured to allow the user to select any of above discussed group (G1, G2, and G3) based on his/her weight and height. Based on the group selected by the user, the proportional control module 110 may convert the pressure applied and released on the foot pedal 120 for generating an appropriate amount of the vacuum level using the vacuum generating unit 122. Thus, the flexibility facilitated by the apparatus 102 for choosing/selecting the values of the plurality of attributes of the various profiles may provide better control and operating confidence to the user.

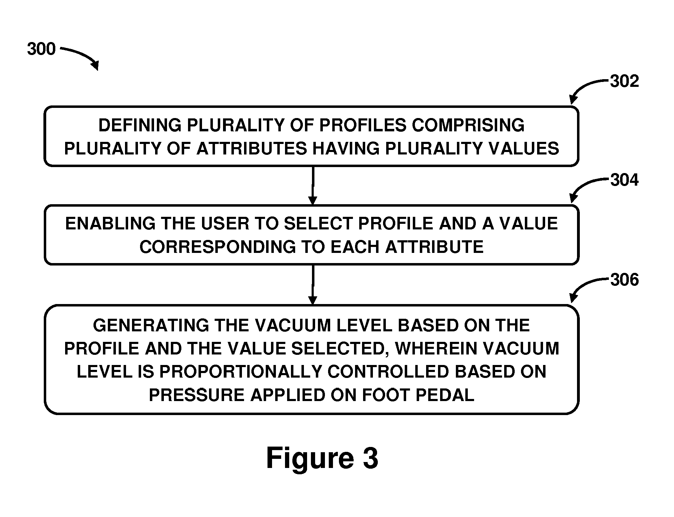

Referring now to Figure 3, method for controlling vacuum level required for oocyte recovery is shown, in accordance with an embodiment of the present disclosure. The method 300 may be described in the general context of computer executable instructions. Generally, computer executable instructions can include routines, programs, objects, components, data structures, procedures, modules, functions, etc., that perform particular functions or implement particular abstract data types. The method 300 may also be practiced in a distributed computing environment where functions are performed by remote processing devices that are linked through a communications network. In a distributed computing environment, computer executable instructions may be located in both local and remote computer storage media, including memory storage devices.

The order in which the method 300 is described is not intended to be construed as a limitation, and any number of the described method blocks can be combined in any order to implement the method 300 or alternate methods. Additionally, individual blocks may be deleted from the method 300 without departing from the spirit and scope of the disclosure described herein. Furthermore, the method can be implemented in any suitable hardware, software, firmware, or combination thereof. However, for ease of explanation, in the embodiments described below, the method 300 may be considered to be implemented in the above described apparatus 102.

At block 302, a plurality of profiles may be defined. Further, each of the plurality of profiles may comprise a plurality of attributes having a plurality of values. According to embodiments, the plurality of attributes may comprise maximum vacuum, pedal force, pedal profile, set temperature, level detect, system theme, and volume level.

At block 304, the user may be enabled to select a profile from the plurality of profiles and a value corresponding to each attribute of the profile selected.

At block 306, a vacuum level may be generated through the vacuum generating unit based upon the profile and the value selected by the user. Further, the vacuum level generated may be proportionally controlled based on a pressure applied on the foot pedal.

Although implementations for the apparatus and its use have been described in language specific to structural features and/or methods, it is to be understood that the appended claims are not necessarily limited to the specific features or methods described. Rather, the specific features and methods are disclosed as examples of implementations for minimizing the risk during the oocyte recovery process.

## Claims

1. An apparatus (102) for controlling a vacuum level required for oocyte recovery from patient's body, wherein the apparatus (102) having a vacuum generating unit (122) and a foot pedal (120), and wherein the apparatus (102) comprises:
a processor (104); and
a memory (106) coupled with the processor (104), wherein the processor (104) executes plurality of modules stored in the memory (106), and wherein the plurality of modules comprising:
a profile control module (112) to define a plurality of profiles, wherein each of the plurality of profiles comprises a plurality of attributes having a plurality of values;
a user interface module (108) to enable a user to select
a profile from the plurality of profiles, and
a value corresponding to each attribute of the profile selected; and
a proportional control module (110) to generate a vacuum level, via a vacuum generating unit, based upon the profile and the value selected, and wherein the vacuum level generated is proportionally controlled based on a pressure applied on a foot pedal.

2. The apparatus (102) of claim 1, wherein the plurality of attributes comprises maximum vacuum, pedal force, pedal profile, set temperature, level detect, system theme, and volume level.

3. The apparatus (102) of claim 1, further comprises a needle (116), a collecting unit (118), and an auxiliary tube (124), wherein
the needle (116), from one end, is inserted into patient's body and connected to the vacuum generating unit (122), from another end, through the auxiliary tube (124) in order to recover an oocyte from the patient's body; and
the collecting unit (118) to collect the oocyte recovered from the patient's body.

4. The apparatus (102) of claim 1, wherein the user is grouped into a predefined category based on weight and height of the user.

5. A method for controlling a vacuum level required for oocyte recovery from patient's body using an apparatus comprising a vacuum generating unit and a foot pedal, and wherein the method comprising:
defining, by a processor, a plurality of profiles, wherein each of the plurality of profiles comprises a plurality of attributes having a plurality of values;
enabling, by the processor, a user to select
a profile from the plurality of profiles, and
a value corresponding to each attribute of the profile selected; and
generating, by the processor, a vacuum level, via a vacuum generating unit, based upon the profile and the value selected, and wherein the vacuum level generated is proportionally controlled based on a pressure applied on a foot pedal.

6. The method of claim 5, wherein the plurality of attributes comprises maximum vacuum, pedal force, pedal profile, set temperature, level detect, system theme, and volume level.
